# EUROPEAN PATENT APPLICATION

(11) **EP 2 213 237 A1**
(43) Date of publication of application: **04.08.2010**
(21) Application number: 09425029.7
(22) Date of filing: 29.01.2009
(51) Int. Cl.: A61B 6/14, A61B 6/00

(54) **X-ray apparatus comprising an angle indicator**

(71) Applicant: Vivi S.r.L., 20124 Milano (IT)
(72) Inventor: Villa, Alfio, 20144 Milano (IT)
(74) Representative: Lunati & Mazzoni S.r.L.

(57) **Abstract**

There is disclosed an X-ray apparatus (1), comprising: an emitting device (2) having an outer shell (11), a support and control device (3) engaging the emitting device (2) and defining a first horizontal axis (5) suitable to allow rotations of the emitting device (2), a counterbalance (6), a pin (10) eccentrically integral with the counterbalance (6) and pivotally engaged with the shell (11), and a viewer (9) and a corresponding indicator element (8) mutually pivoting and selectively engaged with the pin (10) and with the shell (11).

## Description

The present invention relates to an X-ray apparatus comprising an improved emitting device, in particular an emitting device for a dental X-ray device, of the type specified in the preamble of Claim 1.

X-ray apparatuses, in particular for dental X-rays, are currently known.

These comprise a support device which supports an articulated arm which, in turn, supports an emitting device suitable to emit X-rays at the teeth or at a portion of a patient to be X-rayed.

Generally, the support device is constrained to a wall or to a stand and comprises a control unit equipped with control and signalling members, suitable to determine operation of the X-ray apparatus.

The operator using an X-ray apparatus therefore adjusts it through specific controls and places the emitting device in proximity to the mouth, or to the portion of the patient to be X-rayed.

In fact, the emitting device can be positioned, with respect to the support device which remains fixed, both as regards translation movements, due to the degrees of freedom provided by the articulated arm, and as regards angular movements, due to various specific hinges, in particular due to a rotational hinge with horizontal rotation axis positioned between the articulated arm and the emitting device.

The wide possibilities of movement of the emitting device are useful and necessary, but correct positioning of the emitting device is a source of important practical problems.

In fact, angular adjustment of the emitting device must be calibrated with precision in relation to the type of tooth to be X-rayed.

This adjustment is often awkward and complex, even if the patient is placed in the correct position.

In particular, the angular position of the emitting device must be adjusted with respect to the horizontal rotation axis thereof, to adapt the direction of the beam of rays not only to the axis of the tooth to be X-rayed, but also to the position of the X-ray film or sensor on which the image of the tooth is formed.

The X-ray sensor in a great many cases is defined by a plate or the like held in position - as closely as possible in contact with the tooth to be X-rayed - by the patient's fingers, which necessarily place it against the tooth and above all against the gums.

In this situation, it is appropriate for the direction of the beam of rays emitted by the emitting device to take account of the sensible inclination of the sensor with respect to the axis of the tooth.

In fact, it must be noted that the shape and the dimensions of the gums vary for each tooth and as the X-ray sensor is necessarily also placed against the gums, this latter is positioned with varied inclination. Moreover, the inclinations are angularly opposite each other depending on whether the upper or lower dental arch is considered.

If the inclination of the sensor with respect to the tooth to be X-rayed is not taken into account, when the sensor is held by the patient's fingers and also placed against the gums, deformed, irregular and unhomogeneous images are obtained, which can even lead to incorrect diagnoses.

The complexity and awkwardness of adjusting the angular position of the emitting device with respect to its horizontal rotation axis is also due to the fact that in many cases there are no precise references to allow correct positioning.

With the current technique any indications of the angular position - in vertical direction - can therefore only be highlighted in reference to the position of the articulated arm, the position of which is stable in angular direction with respect to a horizontal axis.

The articulated arm is connected pivotally on the emitting device - defining a horizontal rotation axis - at one side or flank of the emitting device and in this area the space available is limited and any indications are partly hidden by the articulated arm.

Moreover, these angular position indications are visible on the opposite side of the emitting device to the position of the person adjusting the X-ray apparatus.

Furthermore, if bulky and voluminous apparatus which are also costly and heavy are to be avoided, the lateral connection of the articulated arm cannot be axially expanded to the other side of the emitting device, where the person who operates it is located, as internally the emitting device is fully engaged by the X-ray emission members.

In this situation the technical aim underlying the present invention is to devise an X-ray apparatus capable of substantially overcoming the aforesaid drawbacks.

Within said technical aim an important object of the invention is to produce an X-ray apparatus which allows the user to precisely adjust the angular position of the emitting device.

Another important object of the invention is to obtain an X-ray device which allows the user to perform simple and immediate adjustment of the angular position of the emitting device.

The technical aim and the objects specified are achieved by an X-ray apparatus according to the appended Claim 1.

Preferred embodiments are highlighted in the dependent claims.

The characteristics and advantages of the invention are better specified below by the detailed description of a preferred embodiment of the invention, with reference to the accompanying drawings, wherein:
**Fig. 1a** schematically shows an operating phase of the apparatus and the angular position thereof with respect to a tooth to be X-rayed;
**Fig. 1b** shows an overall schematic view of a dental X-ray apparatus according to the invention;

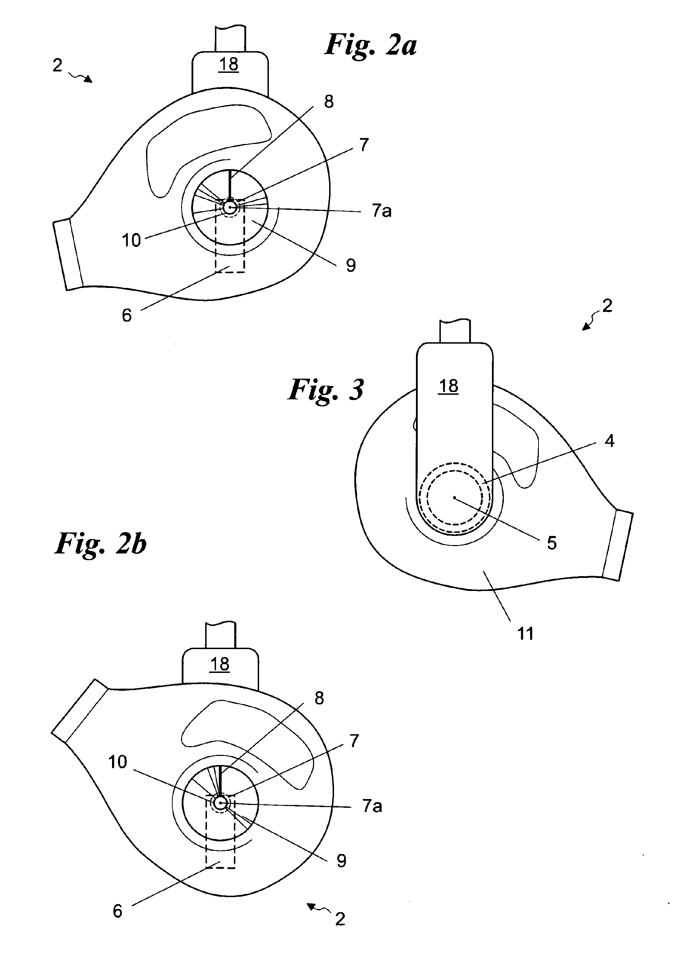

**Fig. 2a** shows a side view of a portion of the X-ray apparatus according to the invention;
**Fig. 2b** shows a side view of the portion of Fig. 2a in a different position;
**Fig. 3** shows a side view, opposite with respect to the views of Figs. 2a and 2b, of the portion of Fig. 2a;
**Fig. 4a** schematically shows a three-dimensional and perspective view of the portion of apparatus of Fig. 2a;
**Fig. 4b** highlights in cross section elements according to the invention placed at the portion of the apparatus of Fig. 2a; and
**Fig. 5** shows a detail of the apparatus according to the invention.

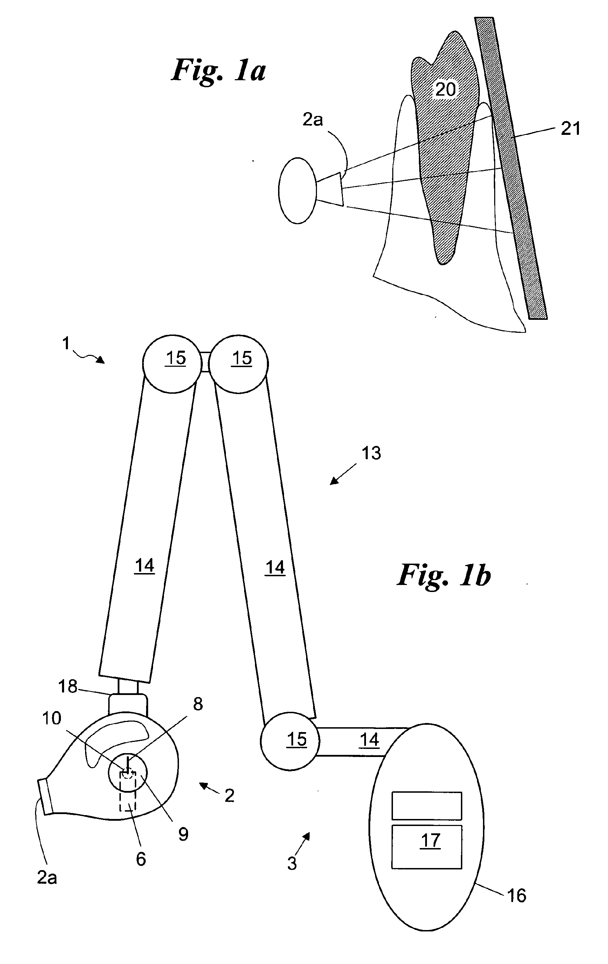

With reference to the figures cited, the X-ray apparatus according to the invention is indicated as a whole with the reference number 1. It comprises, summarily, an emitting device **2**, suitable to emit, through an opening **2a**, a beam of rays having a direction dependent on the position of this emitting device. As shown in Fig. 1a, the beam of rays is directed in particular towards a tooth **20** (in the case shown consisting of a lower premolar) and towards an X-ray sensor **21** located as close as possible to the tooth 20.

The X-ray sensor 21 is in fact placed against the gum **22** and therefore has a specific inclination. To follow this inclination and avoid excessively deformed X-ray images, the beam of rays is also irradiated with a specific inclination, substantially intermediate between that required by the tooth 20 and that required by the X-ray sensor 21.

In detail, a support and control device **3** (Figure 1b) is connected to the emitting device 2 so as to allow positioning thereof, in particular positioning in proximity to a patient, to irradiate him/her and take an X-ray image.

The support and control device 3 comprises an articulated arm **13,** comprising a plurality of arms **14** and articulations **15,** and a support base **16,** comprising control means **17,** such as push buttons and the like, suitable to control and operate said X-ray apparatus 1.

The emitting device 2 is then connected structurally and functionally to the support and control device 3, and is controlled and operated thereby.

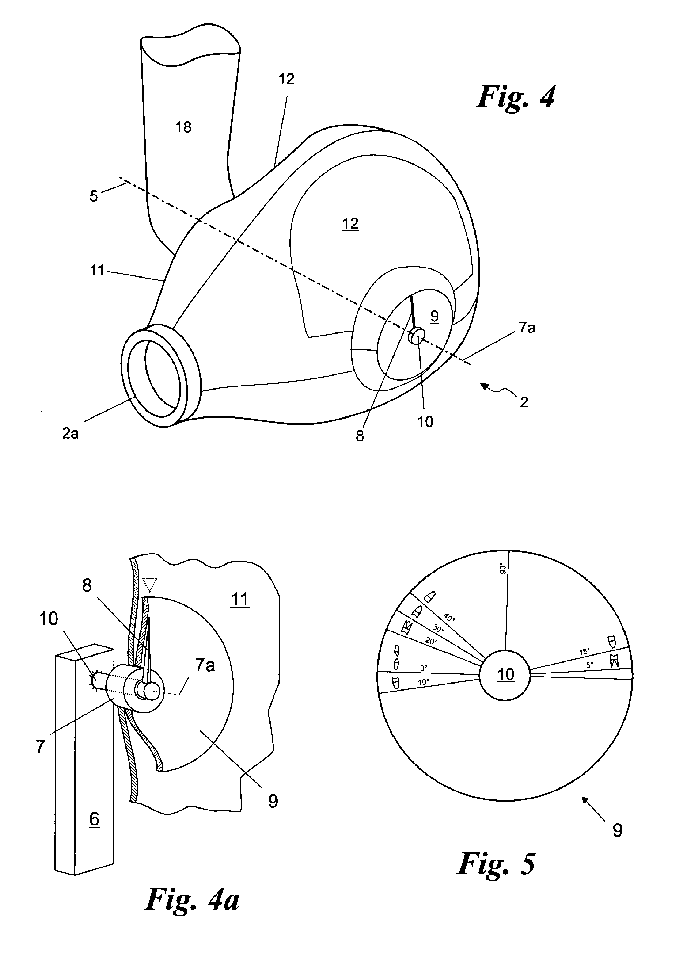

Structurally, the emitting device 2 comprises internal devices and an outer shell **11,** preferable made of polymer material, ellipsoidal in shape and comprising, in the upper part, two concavities **12** symmetrical and substantially defining a handle, as shown in Fig. 4.

The articulated arm 13 preferably ends with a terminal segment **18,** connected to the emitting device 2 and shaped at an angle, with a horizontal end section. The horizontal section connects laterally to the emitting device 2 so as to provide it with a degree of rotational freedom in a horizontal direction.

In the present text the term vertical is intended as the direction of the gravitational gradient, while the term horizontal is intended as the plane perpendicular to the vertical direction.

The terminal segment 18, part of the support and control device 3, is in practice connected to the emitting device 2 by means of a first connection **4** suitable to allow rotation of said emitting device 2 about a first substantially horizontal axis **5**. The first connection 4 is appropriately realized by a rotational hinge concentric with the first axis 5 and engaged with a lateral flank of the outer shell 11.

Internally, the emitting device 2 includes various members suitable to produce said beam of rays. These devices are known and take up the space inside the shell 11, so as to create a compact and manageable body.

Originally, the emitting device 2 also comprises a counterbalance **6** constrained to the emitting device 2 by means of a pin **10** which engages the shell 11 at a second hinge **7**, provided on the lateral face of the shell 11 opposite the one engaged by the support and control device 3. The counterbalance 8 is substantially a flattened rod or plate, made of lead or steel, extending from the pin 10.

The pin 10 and the second hinge 7 define a second axis **7a** substantially horizontal and preferably coincident with the first axis 5.

The counterbalance 6 is eccentric with respect to the pin 10, i.e. it has a centre of mass that does not coincide therewith, and in this situation the second hinge 7 is suitable to allow angular displacement of the counterbalance 6 with respect to the second axis 7a.

In the preferred embodiment the second hinge 7 defines a friction that is substantially null or minimum with the pin 10 and the counterbalance 6 moves freely about the second axis 7a so as to be immediately and spontaneously positioned under the second axis 7A and the first axis 5.

The pin 10 is also, for example, integral with an indicator element **8** on the outside of the shell 11 and substantially realized by a pointer. The indicator element 8 is thus in a position substantially defined by the counterbalance 6.

A viewer or display 9 is provided at the indicator element 8. This viewer 9 is on the outside of the shell 11 and integral therewith when the indicator element 8 is integral with the counterbalance 6.

The viewer 9 is preferably substantially realized by an indicator disk or a goniometer concentric with the pin 10, shown in detail in Fig.5.

It also includes illustrations schematizing the type of tooth which is X-rayed in an optimal manner, in the angular position taken each time by the emitting device 2, in accordance with the indicator element 8.

Operation of the apparatus according to the invention is as follows.

The operator performs the required adjustments using the control means 17 and activates the apparatus 1. Subsequently, he/she moves the emitting device 2 to the portion of the patient to be X-rayed, therefore in particular to the mouth and to a specific tooth.

The operator must then incline the emitting device 2 with respect to the first axis 5, using the concavities 12 defining the grips for a hand, by a specific angle according to the type of tooth to be X-rayed, for example a molar, an incisor, a canine, and to whether it is the upper or lower arch.

In this operation the operator is aided by the viewer or display 9, which indicates the position to be taken with respect to the indicator element 8, which does not change position during rotation of the emitting device 2.

The invention achieves important advantages.

In fact, correct and precise position of the emitting device 2 is ensured by the fact that the angular position thereof is indicated with precision by a pointer whose position is stable as it is regulated by the counterbalance 6.

Moreover, the device 2 is simple and inexpensive, as it is composed of very few parts and of very simple mechanisms. It also has minimum dimensions and can be positioned closely adjacent to the shell 11.

The illustrations on the viewer or display 9, schematizing the type of tooth which is correctly X-rayed, then allow simple and immediate adjustment.

The invention is susceptible to variants.

For example, the counterbalance 6 can either be outside or inside the shell 11.

The viewer 9 can also be provided integral with the pin 10 and with the counterbalance 6, and thus movable with respect to the outer shell 11. In this case the indicator element 8 can be produced directly integral with the outer shell 11 and be realized by a notch or by a ridge or by a graphic element placed in proximity to the viewer 9. Fig. 4a indicates with broken lines a notch indicator integral with the shell 11.

It is therefore the viewer 9 which is in a stable position as a result of the counterbalance and instead the indicator element which rotates together with the shell 11.

In a further variant the emitting device 2 can rotate with minimum friction with respect to the first axis 5 and its angular operating position can be determined by the position of the counterbalance 6, positioned so as to define an appropriate centre of mass, under the first axis 5.

The user only requires to adjust the position of eccentricity of the counterbalance 6 by angularly moving the indicator element 8 integral therewith, or the viewer 9 integral therewith, so that the indicator element 8 is positioned on the correct figure of the viewer 9.

The new eccentric position of the counterbalance 6 must be made stable or fixed, for example by means of clamping by screwing at the pin 10. The stability can also be obtained with friction elements provided at the pin 10.

In this manner the user imposes a rotation of the counterbalance 6 with respect to the hinge 7, repositioning it and defining a new centre of mass of the emitting device 2. The centre of mass of the emitting device 2 is then positioned spontaneously in vertical alignment with respect to the first axis 5, thus in the required operating position.

This variant is also indicated in the figures, as it can be produced by providing a first connection 4 without friction and a second hinge 7 with high friction.

## Claims

1. An X-ray apparatus (1), comprising: an emitting device (2) having an outer shell (11) and suitable to emit a beam of rays, a support and control device (3), connected to said emitting device (2) on one side of said shell (11), provided with a first connection (4) defining a first substantially horizontal rotation axis (5) between said emitting device (2) and said support and control device (3), **characterized in that** it is provided with a counterbalance (6) and a pin (10), eccentrically integral with said counterbalance (6) and pivotally engaging said shell (11), **in that** on the outside of said shell (11) it is provided with an indicator element (8) and a corresponding viewer (9) presenting operating indications of said emitting device (2) which can be selectively highlighted by said indicator element (8), said indicator element (8) and said viewer (9) being mutually pivoting and selectively engaged with said pin (10) and with said shell (11).

2. X-ray apparatus (1) according to claim 1, wherein said counterbalance (6) is provided inside said viewing device (2) and wherein said pin (10) passes through said shell (11).

3. X-ray apparatus (1) according to one or more of the preceding claims, wherein said indicator element (8) is substantially a pointer integral with said pin (10) and wherein said viewer (9) is concentric to said pin (10) and integral with said shell (11).

4. X-ray apparatus (1) according to one or more of the preceding claims, wherein said viewer (9) is integral with said pin (10) and wherein said indicator element (8) is integral with said shell (11) and is placed adjacent to said viewer (9).

5. X-ray apparatus (1) according to one or more of the preceding claims, wherein said viewer (9) is substantially a disk-shaped goniometer concentric with said pin (10).

6. X-ray apparatus (1) according to one or more of the preceding claims, wherein said viewer (9) provides illustrations showing the type of tooth that is preferably X-rayed, in the presence of a corresponding indication of said indicator element (8).

7. X-ray apparatus (1) according to one or more of the preceding claims, wherein said counterbalance (6) is a flattened rod.

8. X-ray apparatus (1) according to one or more of the preceding claims, wherein said pin (10) pivots freely with respect to said shell (11) and wherein said first connection (4) engages said emitting device (2) with friction.

9. X-ray apparatus (1) according to one or more of the preceding claims, wherein said pin (10) pivots with friction with respect to said shell (11) and wherein said first connection (4) engages said emitting device (2) in a freely pivoting manner.

10. X-ray apparatus (1) according to one or more of the preceding claims, wherein said shell (11) comprises concavities( 12) substantially defining a handle suitable to allow operations to move the position of said emitting device.
